# DEMANDE DE BREVET EUROPEEN

(11) **EP 2 308 531 A2**
(43) Date de publication de la demande: **13.04.2011**
(21) Numéro de dépôt: 10370009.2
(22) Date de dépôt: 28.09.2010
(51) Int. Cl.: A61M 5/32, A61B 17/00

(54) **Dispositif de compensation lors du retrait d'une aiguille de chimiothérapie**

(30) Priorité: 29.09.2009 FR 0904644; 30.09.2009 FR 0904665
(71) Demandeur: M2CT (Sarl), 59175 Templemars (FR)
(72) Inventeur: Camus, Michel, 59175 Templemars (FR)

(57) **Abrégé**

Instrument destiné à compenser le phénomène d'aspiration dans une chambre d'oncologie, provoqué par le retrait de l'aiguille en fin de perfusion.

## Description

Lors du retrait d'une aiguille de chimiothérapie, le souci principal du personnel soignant est l'inconvénient que provoque ce retrait. Le phénomène d'aspiration est dû à la masse de l'aiguille qui quitte progressivement la chambre implantée ainsi qu'à la forme coudée de l'aiguille de Huber qui a tendance à augmenter artificiellement le volume de ladite chambre.

Ce mouvement ne peut être trop rapide sous peine de rendre ce geste douloureux, et la délicatesse avec laquelle il est provoqué, permet à l'infirmière d'éviter cette douleur mais complète largement le phénomène d'aspiration à l'extrémité du cathéter, aspiration provoquée par le retrait de l'aiguille de Huber de la chambre dans laquelle celle-ci a été implantée.

Actuellement l'état de l'art, connu et opposé pour ce type de problème, n'est semble-t-il représenté que par le brevet US 2006/015085 de BATES Marc dont le dispositif comporte un matériel de type pistolet utilisant une aiguille incluse dans un cathéter et destiné à la fois à contrôler la profondeur de l'injection et le retrait de l'aiguille pour éventuellement assurer la diffusion du produit injecté et d'autre-part le brevet US 2003/114799 CHEIK ROLAND CHERIF, qui comporte une véritable seringue, prolongée par un dispositif englobant la longueur de l'aiguille qui sera recouverte le moment venu par le prolongement de cette seringue spéciale; tous deux ne présentant pas de similitudes spécifiques avec le produit présenté

Il nous a donc semblé intéressant d'essayer de supprimer différemment ce problème d'aspiration en essayant de compenser ce volume qui disparaît de la chambre.

La compensation de cette aiguille qui quitte la chambre pourrait provenir d'un élément extérieur à celle-ci, tel un liquide, qui injecté dans la chambre, pendant la manoeuvre de retrait de l'aiguille, viendrait donc compenser le volume retiré et ne provoquerait donc pas d'aspiration à l'extrémité du cathéter implanté, source d'ennui majeur pour le patient, telle l'aspiration de sang qui se coagule à l'intérieur dudit cathéter.

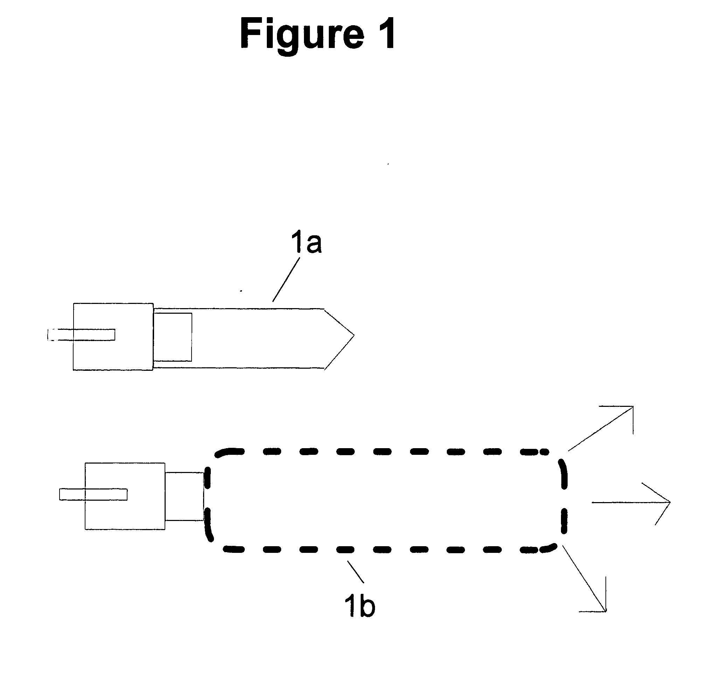

Il pourrait s'agir d'un dispositif tel une poche souple, voire élastique raccordée à un dispositif de raccordement normalisé du genre cône ou verrou américain et dont le volume intérieur serait légèrement supérieur à celui nécessaire à la compensation du phénomène d'aspiration constaté, Cette poche serait cylindrique, ovale ou d'une autre forme {Figure1}géométrique, mais munie d'un dispositif d'action, de par sa nature élastique ou de complément interne ou externe, propre à en assurer la vidange au moment du besoin c'est-à-dire au moment du retrait de l'aiguille.

Et c'est tout d'abord le temps de remplissage de ladite poche suivi du maintien en l'état durant son adaptation au cathéter relié à l'aiguille, au moyen d'un clamp ou d'un robinet.

Cette adaptation étant réalisée , il serait alors possible de permettre la communication de la poche par ouverture du clamp ou du robinet.

Le dispositif d'action réagirait alors normalement pour permettre de vider la poche dans la tubulure. Ce pourrait être la propre déformation de ladite poche induite par son remplissage {1a,1b Figure 1}

Un Matériel dont le réservoir souple est réalisé dans une matière plastique à mémoire de forme { 1a, Figure1} suffisamment rigide de sorte que le réservoir, du fait de son remplissage, se trouve contraint à se dilater et à augmenter de volume intérieur{1b Figure1} mais possède une force mécanique nécessaire et suffisante pour en provoquer la vidange lors de la libération du conduit vers lequel il aura été raccordé, par retour automatique à sa forme initiale{1a Figure1}

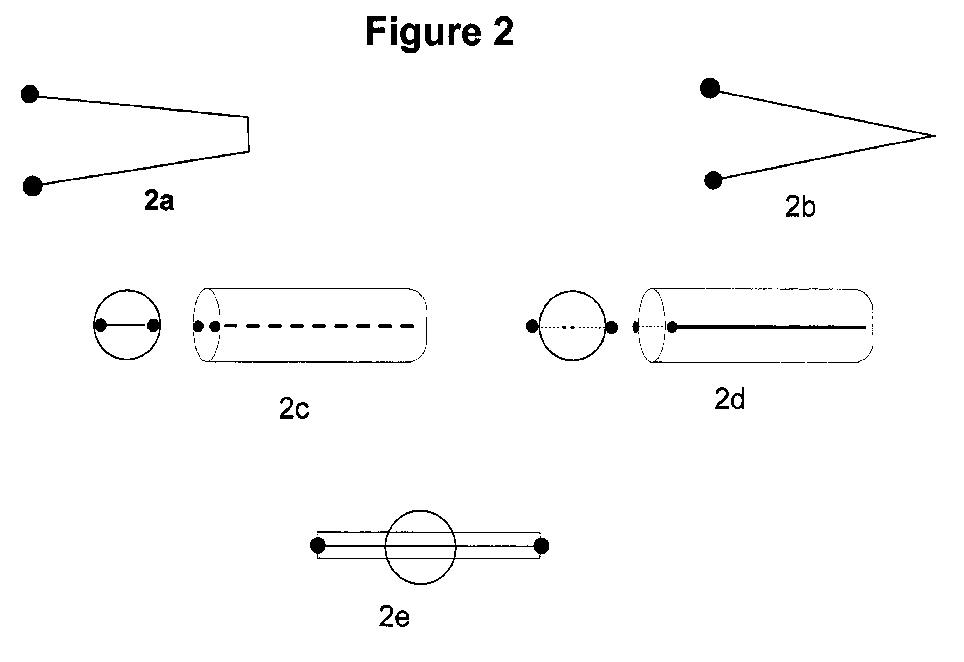

Un ressort de type épingle, placé à l'intérieur mais faisant corps avec la dite poche{2c Figure2}

Cette épingle aurait une forme telle un "U" ouvert {2a Figure2} ou un "V" {2b Figure2} par exemple dont la forme correspondrait à une ouverture plus importante que l'ouverture de la poche

Cette forme par le phénomène du remplissage, de la poche serait donc contrainte par celle-ci et le "U" ou le "V" se refermerait sur lui-même jusqu'au niveau atteint par la quantité injectée dans la poche {2c,2d Figure 2} une fois celle-ci remplie, le robinet ou autre moyen de communication sera alors clos et le dispositif raccordé pendant ce temps à la tubulure d'injection. Ceci, fait, le robinet sera alors ouvert et la libération du conduit de transfert provoquera alors la remise en l'état progressivement du ressort, qui, ayant tendance à aplatir la poche, en permettra sa vidange.{2e Figure 2}tors de l'ouverture de ce conduit après raccordement à la tubulure de vidange.

Un Matériel dont le dispositif consiste en un dispositif d'une forme telle un "U"{2a Figure 2} ou un "V" {2b Figure2}ptacé à l'intérieur {2c Figure2} , se resserrant pendant le remplissage du réservoir {2c, Figure 2}...} et dont la force mécanique permet de provoquer la vidange du réservoir par aplatissement de celui-ci {2e Figure2} lors de l'ouverture du robinet de connexion à la tubulure de raccordement à l'aiguille de chimiothérapie

Un Matériel dont le dispositif consiste en un dispositif d'une forme telle un "U"{2a Figure 2} ou un "V" {2b Figure2} placé à l'extérieur du réservoir{2d Figure2} , se resserrant pendant le remplissage du réservoir {2d Figure 2}...} et dont la force mécanique permet de provoquer la vidange du réservoir par aplatissement de celui-ci {2e Figure2} lors de l'ouverture du robinet de connexion à la tubulure de raccordement à l'aiguille de chimiothérapie

Dans une autre forme on peut envisager : pour un résultat identique,

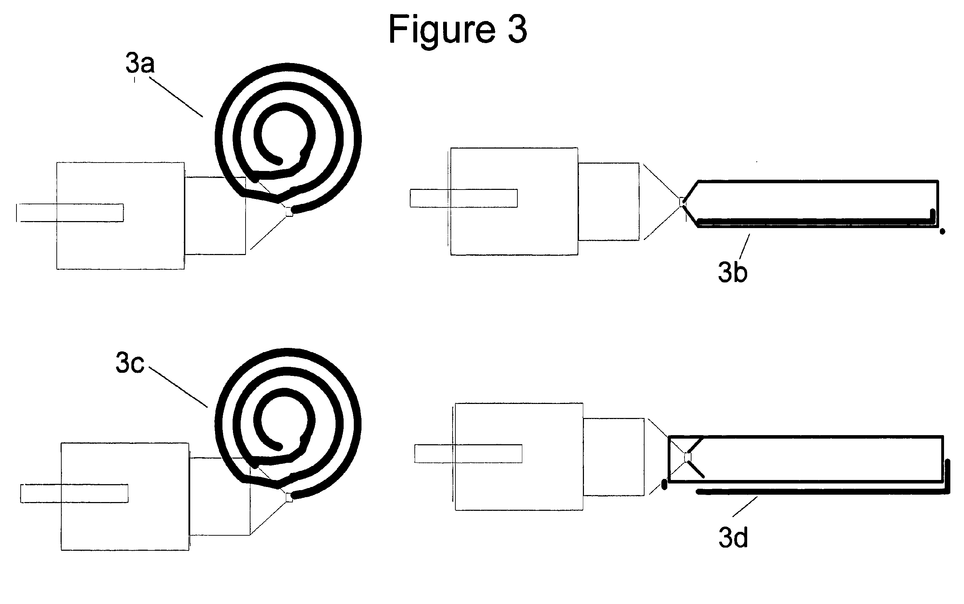

Un matériel composé d'un dispositif d'enroulement sur lui-même placé à l'intérieur {3a,3b Figure 3} du réservoir tel un colimaçon dont la force mécanique permet de provoquer la vidange du réservoir par ré enroulement et écrasement simultané du réservoir {3a, Figure3} après sa déformation initiale du au remplissage de celui-ci , soit lors de l'ouverture du robinet de connexion à la tubulure de raccordement à l'aiguille de chimiothérapie

Un matériel composé d'un dispositif d'enroulement sur lui-même placé à l'extérieur{3c,3d Figure3}.....} du réservoir tel un colimaçon dont la force mécanique permet de provoquer la vidange du réservoir par ré enroulement et écrasement simultané du réservoir i {3c Figure3} après sa déformation initiale du au remplissage de celui-ci , soit lors de l'ouverture du robinet de connexion à la tubulure de raccordement à l'aiguille de chimiothérapie

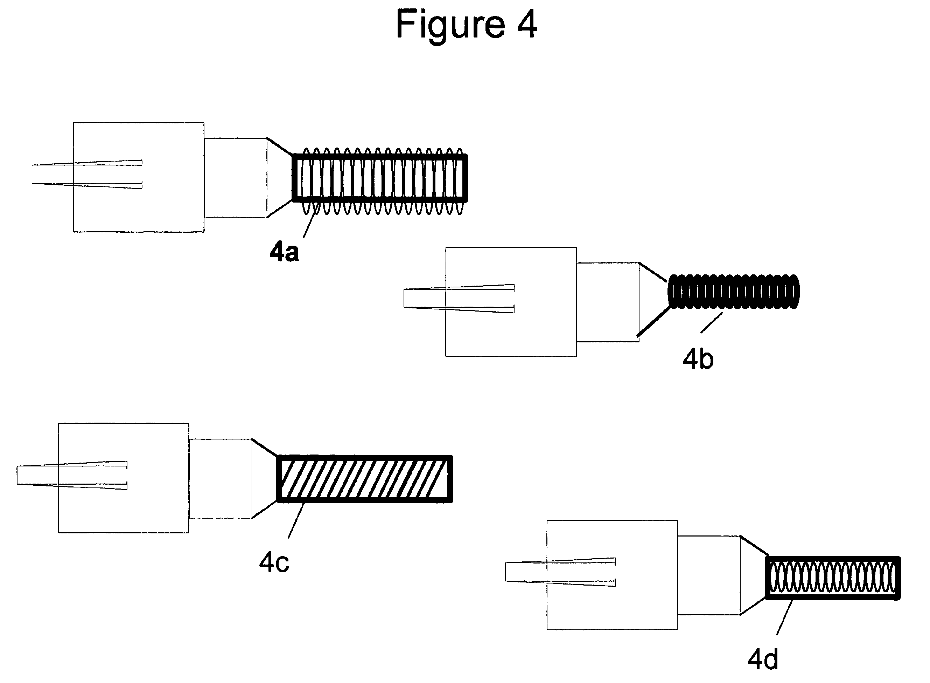

Un dispositif d'expansion tel un ressort enroulé en spirale placé à l'extérieur de la poche qui après s'être déformé lors du remplissage, {4a Figure 4} provoquerait la vidange de ladite poche en revenant à sa position initiale {4b Figure 4} lors de l'ouverture de ce conduit après raccordement à la tubulure de vidange.

Un dispositif d'expansion tel un ressort enroulé en spirale placé à l'intérieur de la poche qui après s'être déformé lors du remplissage, {4c Figure 4} provoquerait la vidange de ladite poche en revenant à sa position initiale {4d Figure 4} lors de l'ouverture de ce conduit après raccordement à la tubulure de vidange.

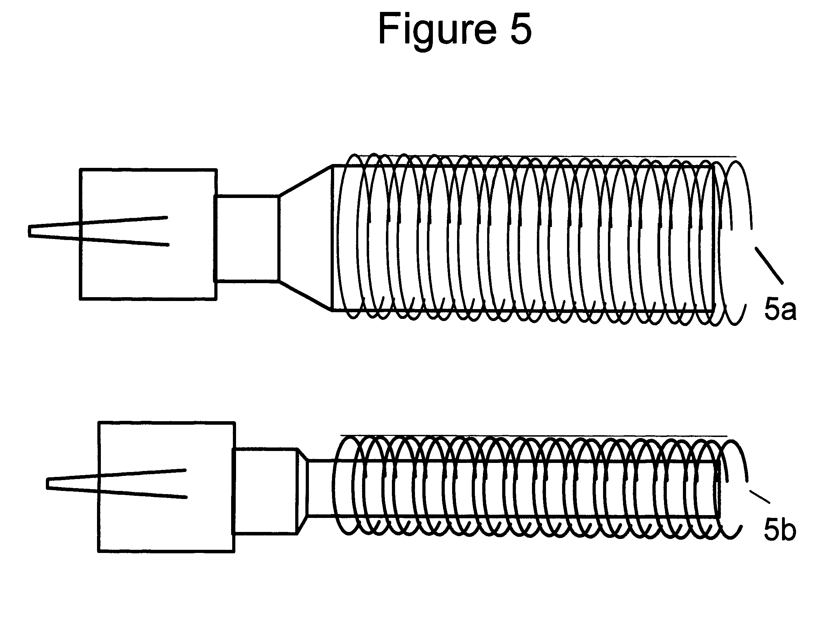

Un dispositif d'expansion tel une succession de cerclips relié entre-eux ou non, qui se trouveraient contraints de s'ouvrir lors du remplissage du dispositif {5a Figure5} pour se refermer ensuite lors de l'ouverture du conduit après raccordement à la tubulure de vidange {5b Figure 5 }

Un dispositif d'hémi cercles successifs tels une succession de cerclips reliés ou non entre-eux placés à l'extérieur du réservoir.{ Figure5}, mais dont la déformation permettra au réservoir de se remplir par la libération de la contrainte dudit dispositif {5a Figure5} mais qui reprendra sa forme initiale resserrée {5b Figure5}après ouverture du robinet de connexion à la tubulure de raccordement à l'aiguille de chimiothérapie

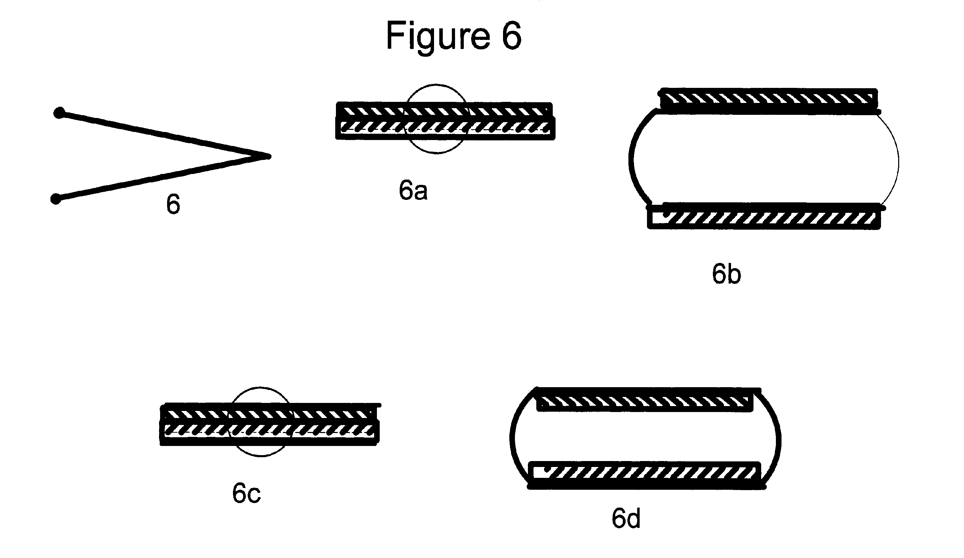

Un dispositif qui sera, lors de sa fabrication, rendu solidaire d'un dispositif externe plat, tel un bec de canard, {6 Figure6}propre à lui infliger un aplatissement {6a Figure6} tout en lui permettant de reprendre sa forme initiale de réservoir souple lors de son remplissage{6b Figure6} ; cet aplatissement progressif provoquant donc la vidange du dispositif dans la tubulure de raccordement à l'aiguille de chimiothérapie.

Un dispositif qui sera, lors de sa fabrication, rendu solidaire d'un dispositif interne plat, tel un bec de canard, {6 Figure6}propre à lui infliger un aplatissement {6c Figure6} tout en lui permettant de reprendre sa forme initiale de réservoir souple lors de son remplissage{6d Figure6} ; cet aplatissement progressif provoquant donc la vidange du dispositif dans la tubulure de raccordement à l'aiguille de chimiothérapie.

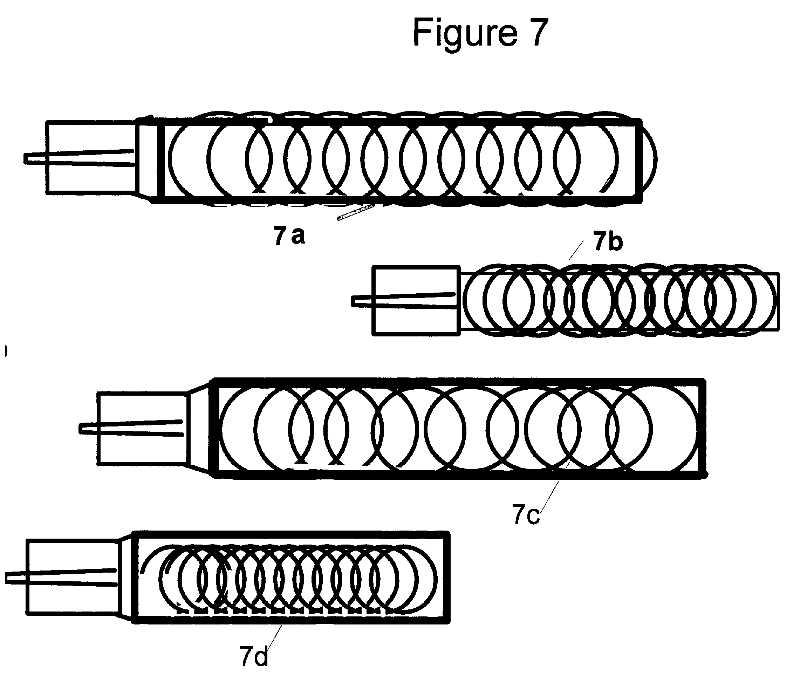

Un dispositif externe constitué d'un ressort{7a Figure 7} s'allongeant d'un mouvement linéaire lors du remplissage du réservoir tout en lui permettant de reprendre sa forme initiale,{7b Figure 7} grâce à l'effet ressort ce qui provoquera donc, le moment venu, la vidange du dispositif, par récupération du volume interne, dans la tubulure de raccordement à l'aiguille de chimiothérapie

Un dispositif interne constitué d'un ressort{7c Figure 7} s'allongeant d'un mouvement linéaire lors du remplissage du réservoir tout en lui permettant de reprendre sa forme initiale,{7d Figure 7} grâce à l'effet ressort ce qui provoquera donc, le moment venu, la vidange du dispositif, par récupération du volume interne, dans la tubulure de raccordement à l'aiguille de chimiothérapie

## Revendications

1. Matériel dit à pression positive conçu pour permettre d'éviter le phénomène d'aspiration lors du retrait d'une aiguille de chimiothérapie **caractérisé en ce qu'**il consiste en un réservoir souple voire élastique, d'un volume nécessaire et suffisant pour compenser le volume aspiré lors du retrait de l'aiguille de la chambre de chimiothérapie, réservoir destiné à recevoir du liquide par l'intermédiaire d'un élément de remplissage tel une seringue, via un élément de raccordement standard type cône ou verrou américain, ledit réservoir souple étant, lors de sa fabrication, pourvu d'un dispositif propre ou auxiliaire possédant une force mécanique pour, après déformation dudit réservoir et par conséquent augmentation de son volume, induite par son remplissage, donc par modification ou extension de sa forme initiale {1a,1b Figure1}, provoquer le retour progressivement à sa position de départ, c'est-à-dire un réservoir avec un volume interne quasiment nul du à la vidange dudit réservoir dans la tubulure à laquelle il aura été raccordé

2. Matériel selon la revendication 1 **caractérisé en ce que** le réservoir souple est réalisé dans une matière plastique à mémoire de forme { 1a, Figure1} suffisamment rigide de sorte que le réservoir, du fait de son remplissage, se trouve contraint à se dilater et à augmenter de volume intérieur{1b Figure1} mais possède une force mécanique nécessaire et suffisante pour en provoquer la vidange lors de la libération du conduit vers lequel il aura été raccordé, par retour automatique à sa forme initiale{1a Figure1}

3. Matériel selon la revendication 1 **caractérisé en ce que** le dispositif consiste en un dispositif d'une forme telle un "U"{2a Figure 2} ou un "V" {2b Figure2}ptacé à l'intérieur {2c Figure2} du réservoir, se resserrant pendant le remplissage du réservoir {2c, Figure 2}...} et dont la force mécanique permet de provoquer la vidange du réservoir par aplatissement de celui-ci {2e Figure2} lors de l'ouverture du robinet de connexion à la tubulure de raccordement à l'aiguille de chimiothérapie.

4. Matériel selon la revendication 1 **caractérisé en ce que** le dispositif consiste en un dispositif d'une forme telle un "U"{2a Figure 2} ou un "V" {2b Figure2}placé à l'extérieur du réservoir{2d Figure2} , se resserrant pendant le remplissage du réservoir {2d Figure 2}...} et dont la force mécanique permet de provoquer la vidange du réservoir par aplatissement de celui-ci {2e Figure2} lors de l'ouverture du robinet de connexion à la tubulure de raccordement à l'aiguille de chimiothérapie.

5. Matériel selon la revendication 1 **caractérisé en ce que** le dispositif consiste en un dispositif d'enroulement sur lui-même placé à l'intérieur {3a,3b Figure 3} du réservoir tel un colimaçon dont la force mécanique permet de provoquer la vidange du réservoir par ré enroulement et écrasement simultané du réservoir {3a Figure3} après sa déformation initiale , soit lors de l'ouverture du robinet de connexion à la tubulure de raccordement à l'aiguille de chimiothérapie

6. Matériel selon la revendication 1 **caractérisé en ce que** le dispositif consiste en un dispositif d'enroulement sur lui-même placé à l'extérieur{3c,3d Figure3}.....} du réservoir tel un colimaçon dont la force mécanique permet de provoquer la vidange du réservoir par ré enroulement et écrasement simultané du réservoir {,3c Figure3} après sa déformation initiale , soit lors de l'ouverture du robinet de connexion à la tubulure de raccordement à l'aiguille de chimiothérapie

7. Matériel selon la revendication 1 **caractérisé en ce que** le dispositif consiste en un dispositif spiralé placé à l'extérieur dont la déformation permettra au réservoir de se remplir par la libération de la contrainte dudit dispositif spiralé{4a Figure4} mais qui reprendra sa forme initiale resserrée {4b Figure4}après ouverture du robinet de connexion à la tubulure de raccordement à l'aiguille de chimiothérapie

8. Matériel selon la revendication 1 **caractérisé en ce que** le dispositif consiste en un dispositif spiralé placé à lintérieur dont la déformation permettra au réservoir de se remplir par la libération de la contrainte dudit dispositif spiralé{4c Figure4} mais qui reprendra sa forme initiale resserrée {4d Figure4}après ouverture du robinet de connexion à la tubulure de raccordement à l'aiguille de chimiothérapie

9. Matériel selon la revendication 1 **caractérisé en ce que** le dispositif consiste en un dispositif d'hémi cercles successifs tels une succession de cerclips reliés ou non entre-eux placés à l'extérieur du réservoir. {Figure5}, mais dont la déformation permettra au réservoir de se remplir par la libération de la contrainte dudit dispositif {5a Figure5} mais qui reprendra sa forme initiale resserrée {5b Figure5}après ouverture du robinet de connexion à la tubulure de raccordement à l'aiguille de chimiothérapie

10. Matériel selon la revendication 1 **caractérisé en ce que** le dispositif consiste en un dispositif d'hémi cercles successifs tels une succession de cerclips reliés ou non entre-eux placés à l'intérieur du réservoir.{ Figure5}, mais dont la déformation permettra au réservoir de se remplir par la libération de la contrainte dudit dispositif {5c Figure5} mais qui reprendra sa forme initiale resserrée {5d Figure5}après ouverture du robinet de connexion à la tubulure de raccordement à l'aiguille de chimiothérapie

11. Matériel selon la revendication 1 **caractérisé en ce que** le dispositif sera, lors de sa fabrication, rendu solidaire d'un dispositif externe plat, tel un bec de canard, {6 Figure6}propre à lui infliger un aplatissement {6a Figure6} tout en lui permettant de reprendre sa forme initiale de réservoir souple lors de son remplissage{6b Figure6} ; cet aplatissement progressif provoquant donc la vidange du dispositif dans la tubulure de raccordement à l'aiguille de chimiothérapie.

12. Matériel selon la revendication 1 **caractérisé en ce que** le dispositif sera, lors de sa fabrication, rendu solidaire d'un dispositif interne plat, tel un bec de canard, {6 Figure6}propre à lui infliger un aplatissement {6c Figure6} tout en lui permettant de reprendre sa forme initiale de réservoir souple lors de son remplissage{6d Figure6} ; cet aplatissement progressif provoquant donc la vidange du dispositif dans la tubulure de raccordement à l'aiguille de chimiothérapie.

13. Matériel selon la revendication 1 **caractérisé en ce que** le dispositif sera, lors de sa fabrication, rendu solidaire d'un dispositif externe constitué d'un ressort{7a Figure 7} s'allongeant d'un mouvement linéaire lors du remplissage du réservoir tout en lui permettant de reprendre sa forme initiale,{7b Figure 7} grâce à l'effet ressort ce qui provoquera donc, le moment venu, la vidange du dispositif, par récupération du volume interne, dans la tubulure de raccordement à l'aiguille de chimiothérapie

14. Matériel selon la revendication 1 **caractérisé en ce que** le dispositif sera, lors de sa fabrication, rendu solidaire d'un dispositif interne, constitué d'un ressort{7c Figure 7} s'allongeant d'un mouvement linéaire lors du remplissage du réservoir tout en lui permettant de reprendre sa forme initiale,{7d Figure 7} grâce à l'effet ressort ce qui provoquera donc, le moment venu, la vidange du dispositif, par récupération du volume interne, dans la tubulure de raccordement à l'aiguille de chimiothérapie
